# EUROPEAN PATENT SPECIFICATION

(11) **EP 3 164 155 B1**
(45) Date of publication and mention of the grant of the patent: **09.02.2022**
(21) Application number: 15731463.4
(22) Date of filing: 12.06.2015
(51) Int. Cl.: A61K 47/32, A61K 9/14, A61K 31/557, A61K 9/10, A61P 9/12

(54) **TREPROSTINIL FORMULATIONS**
TREPROSTINILFORMULIERUNGEN
FORMULATIONS DE TRÉPROSTINIL

(30) Priority: 13.06.2014 US 201462011689 P
(43) Date of publication of application: 10.05.2017
(73) Proprietor: United Therapeutics Corporation, Silver Spring, MD 20910 (US)
(72) Inventor: PHARES, Ken, Hillborough, North Carolina 27278 (US); CHANG, Courtney, Raleigh, North Carolina 27617 (US)
(74) Representative: Hoffmann Eitle
(86) International application number: PCT/US2015/035595
(87) International publication number: WO 2015/192030

(56) References cited:
- WO-A1-2009/048606
- WO-A1-2009/137066
- WO-A1-2009/158010
- Anonymous: "Oral controlled release formulation design and drug delivery: Theory to practice", 2010, John Wiley&Sons, Inc., XP002743380, pages 7-8

## Description

### FIELD

The present disclosure relates to ion-exchange resin complexes with prostacyclin derivatives, such as treprostinil.

### SUMMARY

One embodiment is a composition comprising a) treprostinil or a pharmaceutically acceptable salt thereof and b) an anion ion exchange resin, which is preferably in the form of an ion complex.

Another embodiment is a pharmaceutical formulation comprising such ion complex of treprostinil or a pharmaceutically acceptable salt thereof and a pharmaceutically acceptable carrier.

Yet another embodiment is a method of preparing a treprostinil formulation comprising admixing an ion-exchange resin and a solution comprising treprostinil or a pharmaceutically acceptable salt thereof to form a suspension comprising an ion complex of treprostinil or its derivative and the ion-exchange resin. The treprostinil/ion-exchange resin preferably forms a resonate that is coated with a functional coat to slow the release of treprostinil during dissolution. The coated resonate may be formulated as a suspension for the final dosage form.

### FIGURES

Figure 1 shows UV absorbance spectra of treprostinil diolamine and calls out the optical density (OD) of treprostinil diethanolamine at 228 nm (2.563) and 270 nm (0.757). The spectrophotometer generates the data as raw data, followed by blank corrected spectrum, corrected for the solvent. The average spectrum is an average of two spectra corrected for blank. The average spectrum is used for calculating the concentration.
Figures 2A-B show the optical density of treprostinil at 228 nm (A) and 270 nm (B) as a function of treprostinil concentration.
Figure 3 shows photographs of PUROLITE^{™} (A) and DUOLITE^{™} (B) resins before being complexed with treprostinil diolamine.
Figure 4 shows a concentration of unbound treprostinil diolamine as a function of time when treprostinil diolamine is mixed with PUROLITE^{™} resin at a treprostinil diolamine:resin ratio of 1:1 (w/w).
Figure 5A shows i) treprostinil diolamine loading per gram of PUROLITE^{™} resin and ii) treprostinil diolamine loading efficiency during PUROLITE^{™} resin and treprostinil complexation. Figure 5B is a photograph of PUROLITE^{™} and treprostinil complexes.
Figure 6A shows i) treprostinil loading per gram of DUOLITE^{™} resin and ii) treprostinil diolamine loading efficiency during DUOLITE^{™} resin and treprostinil complexation. Figure 6B is a photograph of DUOLITE^{™} and treprostinil complexes.

### DETAILED DESCRIPTION

Unless otherwise specified "a" or "an" means one or more.

Treprostinil has been described in U.S. Patent No. 4,306,075. Treprostinil, and other prostacyclin derivatives, may be prepared as described in Moriarty, et al in J. Org. Chem. 2004, 69, 1890-1902, Drug of the Future, 2001, 26(4), 364-374, U.S. Patent Nos. 6,441,245, 6,528,688, 6,700,025, 6,809,223, 6,756,117, 8,461,393, 8,481,782, 8,242,305, 8,497,393, 8,748,657, and 8,940,930, U.S. Published Patent Application Nos. 2012/0190888 and 2012/0197041, and PCT Publication No. WO2012/009816.

Various uses and/ or various forms of treprostinil are disclosed, for examples, in U.S. Patent Nos. 5,153,222, 5,234,953, 6,521,212, 6,756,033, 6,803,386, 7,199,157, 6,054,486, 7,417,070, 7,384,978, 7,879,909, 8,563,614, 8,252,839, 8,536,363, 8,410,169, 8,232,316, 8,609,728, 8,350,079, 8,349,892, 7,999,007, 8,658,694, 8,653,137, 8,747,897, and 8,969,409, U.S. Published Patent Application Nos. 2005/0165111, 2009/0036465, 2008/0200449, 2010/0076083, 2012/0216801, 2008/0280986, 2009/0124697, 2013/0261187, 2014/0275262, 2014/0275616, and 2014/0288314, and PCT Publication No. WO00/57701. WO 2009/048606 A1 discloses a non-aqueous extrudable composition comprising at least one thermoplastic polymer where treprostinil is one of the active agents mentioned.

The present invention relates to complexes of anionic ion exchange resins with treprostinil or a pharmaceutically acceptable salt thereof.

Remodulin^{®}, Tyvaso^{®} and Orenitram^{™} all contain treprostinil as the active ingredient and have been approved by the U.S. Food and Drug Administration for the treatment of pulmonary arterial hypertension (PAH). Remodulin^{®} treprostinil injection is administered either subcutaneously or intravenously. Tyvaso^{®} treprostinil inhalation solution is administered by inhalation using a nebulizer. Orenitram^{®} treprostinil extended release tablets are administered orally.

Treprostinil is known by the chemical name 2-((1R,2R,3aS,9aS)-2-hydroxy-1-((S)-3-hydroxyoctyl)-2,3,3a,4,9,9a-hexahydro-1H-cyclope nta[b]naphthalen-5-yloxy)acetic acid and has the following structure: 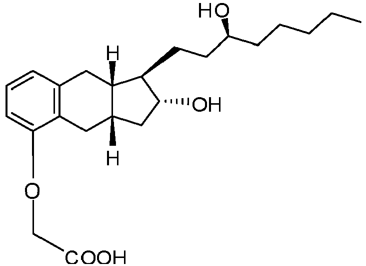 Treprostinil has a net negative charge in solution at pH values 2 pH units above its pKa at its carboxylate moiety. This negative charge can be used to form an ion complex with an anionic ion-exchange resin.

An ion complex with an anionic ion-exchange resin also may be formed by a treprostinil derivative having a carboxylate moiety. Such treprostinil derivatives may be one or more treprostinil esters, such as those disclosed in U.S. Patent No. 7,417,070. The treprostinil derivative may also be a "pegylated" treprostinil, i.e., treprostinil linked to polyethylene glycol, such as pegylated treprostinil forms disclosed in PCT Publication No. WO00/57701 and U.S. Published Patent Application No. 2014/0288314. Unless otherwise specified, "treprostinil," as used herein, includes free acid treprostinil, pharmaceutically acceptable salts of treprostinil, treprostinil derivatives, and pharmaceutically acceptable salts of treprostinil derivatives.

In some embodiments, a pharmaceutically acceptable salt of treprostinil is used. "Pharmaceutically acceptable salts" mean salts that are pharmaceutically acceptable, and which possess the desired pharmacological activity. A pharmaceutically acceptable salt may be a pharmaceutically acceptable organic or inorganic base salt of treprostinil. Representative pharmaceutically acceptable salts include, *e*.*g*., alkali metal salts, alkali earth salts, ammonium salts. Pharmaceutically acceptable salts can include base addition salts. Base addition salts of treprostinil may be formed with sodium, ammonia, potassium, calcium, barium, lithium, magnesium, cesium, ethanolamine, diethanolamine, N-methylglucamine, tromethamine, choline, L-lysine, L-arginine and the like. Salts of treprostinil are disclosed, for example, in U.S. Patent No. 7,417,070 and U.S. Published Patent Application No. 2014/0275616.

As used herein, the term "treprostinil-ion-exchange resin complex" refers to an ion complex formed by a) treprostinil or a pharmaceutically acceptable salt of treprostinil and b) an ion-exchange resin, such as an anion ion-exchange resin.

A number of anion resins may be used for forming complexes with treprostinil and its derivatives. Suitable ion-exchange resins may be water-insoluble and may comprise an organic and/or inorganic matrix containing functional groups that are ionic or capable of being ionized under the appropriate conditions of pH. The ion-exchange resin may be pharmacologically inert. The organic matrix may be synthetic (e.g., polymers or copolymers of acrylic acid, methacrylic acid, sulfonated styrene, sulfonated divinylbenzene), or partially synthetic (e.g. modified cellulose and dextrans). The inorganic matrix may comprise silica gel modified by the addition of ionic groups. For anion resins, covalently bound ionic groups may be strongly basic (e.g., quaternary amino groups, such as trimethylammonium groups, poly (acrylamido-N-propyltrimethylammonium chloride (polyAPTAC), or poly[(3-(methacryloylamino)-propyl] trimethylammonium chloride (PolyMAPTAC)). Anion resins may also be weakly basic (e.g., primary, secondary, and/or ternary amino groups, including polyethylene amine). Pharmaceutical applications of ion-exchange resins are disclosed, for example, in Pande S.V., M. D. Kshirsagar, and A. V. Chandewar, International Journal of Advances in Pharmaceutical Sciences 2. 2.1 (2011): 8-16. In general, the types of ion-exchangers suitable for use in ion-exchange chromatography and for applications, such as deionization of water, are suitable for use in the controlled release of drug preparations. Such ion-exchangers are described, for example, by H. F. Walton in "Principles of Ion Exchange" (pp: 312-343) and "Techniques and Applications of Ion-Exchange Chromatography" (pp: 344-361) in Chromatography. (E. Heftmann, editor), van Nostrand Reinhold Company, New York (1975). Ion-exchange resins that can be used may have exchange capacities of about 6 milliequivalents (meq)/gram and preferably about 5.5 meq/gram or below. In some embodiments, the ion-exchange resin will have an exchange capacity of 5 meq/gram or below, 4.5 meq/gram or below, 4 meq/gram or below, 3.5 meq/gram or below, or 3 meq/gram or below. In some embodiments, the ion exchange resin will have an exchange capacity of 0.5 meq/gram to 4 meq/gram, 1.0 meq/gram to 3 meq/gram, 1.5 meq/gram to 2.5 meq/gram, or 1.8 meq/gram to 2.2 meq/gram.

Typically, the size of the ion-exchange resin particles is from about 5 microns to about 1000 microns. The size of the ion-exchange resin particles in some embodiments can be from about 10 microns to about 750 microns, 20 microns to 500 microns, 50 microns to 400 microns, 50 mcirons to 200 microns, and from 75 microns to 125 microns. In some emdodiments, the size of the ion-exchange resin particles can be about 100 microns. In one embodiment, the particles used in liquid dosage form can be within the range of about 40 microns to about 250 microns for liquid dosage forms. In some embodiments, the particles used in a solid dosage form composition, e.g., tablets and capsules, can be 1,000 micron or less. Particle sizes substantially below the lower limit may be difficult to handle in all steps of the processing. Generally, uncoated drug-ion exchange resin particles may tend to be at the lower end of these ranges, whereas coated drug-ion exchange resin particles may tend to be at the higher end of these ranges. However, both uncoated and coated drug-ion exchange resin particles may be designed within these size ranges.

Both regularly and irregularly shaped particles may be used as resins. Regularly shaped particles are those particles that substantially conform to geometric shapes such as spherical, elliptical, cylindrical and the like. Irregularly shaped particles are all particles not considered to be regularly shaped, such as particles with amorphous shapes and particles with increased surface areas due to surface channels or distortions.

One example of an anion ion-exchange resin is a cholestyramine resin, a strong base type 1 anion ion-exchange resin powder with a polystyrene matrix and quarternary ammonium functional groups. The exchangeable anion is generally chloride, which can be exchanged for, or replaced by, another anionic species. A commercially available cholestyramine resin is PUROLITE^{™} A430MR resin. As described by its manufacturer, Purolite, this resin has a mean typical particle size of less than 150 microns, a pH in the range of 4-6, and an exchange capacity of 1.8-2.2 meq/dry gm. Another pharmaceutical grade cholestyramine resin is available from Rohm and Haas as DUOLITE^{™} AP143/1094. DUOLITE^{™} AP143/1094 is described by the manufacturer as having a particle size in the range of 95%, less than 100 microns, and 40%, less than 50 microns. DUOLITE^{™} AP143/1094 is also described by the manufacturer as having a pH of 4.0 to 6.0 (slurry value), 13.0% to 14.0% chloride content, and 1.8-2.2 meq/dry gm of sodium glycholate exchange capacity. The commercial literature from the suppliers of these and other resins is incorporated herein by reference (PUROLITE A-430 MR; DOW Cholestryramine USP, Form No. 177-01877-204, Dow Chemical Company; DUOLITE AP143/1083, Rohm and Haas Company, IE-566EDS--February 06).

In some embodiments, the anion ion-exchange resin may be a polyamine-based resin. For example, in some embodiments, a polyamine-based resin utilizing an acrylic or polyacrylic matrix, such as polyaminoacrylamide, can be used. One example of a polyamine-based resin is PUROLITE^{™} A830EMR.

In many embodiments, the anion ion-exchange resin may be a bile acid sequestrant or resin. Examples of bile acid sequestrants include cholestyramine (trade names Questran, Questran Light, Cholybar, Olestyr), colestipol (trade names Colestid, Cholestabyl), colesevelam (marketed as Cholestagel in Europe and Welchol in the USA). Bile acid sequestrants are disclosed, for example, in U.S. Patent Nos. 4,093,657, 4,185,088, 4,593,073, 5,091,175, 5,491,397, 5,607,669, 5,679,717, 5,693,675, 5,917,007, 5,919,832, 5,929,184, 6,066,678, 6,129,910, 6,190,649, 6,203,785, 6,271,264, 6,294,163, 6,433,026, 6,517,825, 6,610,283, 6,784,254, 7,101,916, 7,125,547, and 7,229,613 and U.S. Published Patent Application Nos. 2004/0151687, 2007/0098678, 2007/0122375, 2007/0190021, 2008/0261942, 2010/0179235, 2011/0152204, 2013/0022570, and 2013/189215.

In some embodiments, a composition comprising treprostinil and an ion-exchange resin may be prepared by admixing the ion-exchange resin, such as a cholestyramine resin, and a solution, such as an aqueous solution, of treprostinil, such as a pharmaceutically acceptable salt of treprostinil, to form a suspension or dispersion comprising an ion complex of treprostinil or its derivative and the ion-exchange resin. Similarly, in some embodiments, a composition comprising treprostinil and an ion-exchange resin may be prepared by admixing a solution, such as an aqueous solution, of treprostinil, such as a pharmaceutically acceptable salt of treprostinil and the ion-exchange resin, such as a cholestyramine resin to form a suspension or dispersion comprising an ion exchange complex of treprostinil or its derivative and the ion exchange resin. This suspension then may be stirred, mixed, or otherwise agitated for at least 15 minutes, at least 30 minutes, at least 45 minutes, at least 60 minutes, at least 75 minutes, at least 90 minutes, at least 120 minutes, at least 150 minutes, at least 180 minutes, at least 210 minutes, at least 240 minutes, at least 270 minutes, or at least 360 minutes. Upon stirring, mixing, or otherwise agitating, the resulting suspension or dispersion may be filtered. Filtration separates the resonate from any free treprostinil, leaving only the resonate as a solid. The filter pore size can be as small as about 1 micron diameter since the resin initially is about 100 microns.

Binding of treprostinil to the ion-exchange resin may be accomplished using methods known in the art. Anion ion-exchange resins are available in either Cl- or OH-forms. Thus, binding reactions for an acidic drug, such as treprostinil (including its derivative), to an anion exchange resin may be as follows: (a) resin (if in Cl- form ) plus drug (in salt form); (b) resin (if in Cl- form) plus drug (as free acid); (c) resin (in OH-) plus drug (in salt form); (d) resin (in OH-) plus drug (as free acid). All of these reactions except (d) have ionic by-products and the anions generated when the reactions occur compete with the anionic drug for binding sites on the resin. This typically results in reduced levels of drug bound to the ion exchange resin at equilibrium. For acidic drugs, stoichiometric binding of drug to resin may be accomplished through reaction (d). The binding may be performed, for example, as a batch or column process, as is known in the art.

Preferably, the drug-ion exchange resin complex thus formed is collected by filtration and washed with appropriate solvents to remove unbound drug or by-products. The complexes can be air-dried in trays, in a fluid bed dryer, microwave, or other suitable dryer, at room temperature or at elevated temperature or under vacuum.

In some embodiments of preparing the complexes, batch equilibration is used when loading a drug, such as treprostinil, into ion exchange resins of distinct particle size. The total ion exchange capacity represents the maximum achievable capacity for exchanging cations or anions measured under ideal laboratory conditions. The capacity that will be realized when loading a drug onto ion exchange resin will be influenced by such factors as the inherent selectivity of the ion exchange resin for the drug, the drug's concentration in the loading solution, and the concentration of competing ions also present in the loading solution. The rate of loading will be affected by the activity of the drug and its molecular dimensions as well as the extent to which the polymer phase is swollen during loading.

When utilizing a batch or equilibrium process for loading a drug onto an ion exchange resin, it may be desirable to load as much as possible of the drug onto the ion exchange resin. Complete transfer of the drug from the loading solution is not likely in a single equilibrium stage. Accordingly, more than one equilibration may be required in order to achieve the desired loading onto the ion exchange resin. The use of two or more loading stages, separating the resin from the liquid phase between stages, is a means of achieving maximum loading of the drug onto the ion exchange resin although loss of drug from the liquid phase of the final stage occurs.

The amount of treprostinil that may be loaded onto an ion-exchange resin may range from 1% to 75% or from 2% to 70 % or from 3% to 60% or from 5% to 50% or from 10 % to 40% or a subrange within these ranges by weight of the treprostinil-ion exchange resin particles. Loading is the amount of treprostinil per amount of resin, and treprostinil can be loaded for example in an amount of 1.6 g of treprostinil per gram of resin.

In some embodiments, an ion exchange resin may be a cholestyramine resin, such as DUOLITE^{™} or PUROLITE^{™} resin, and a treprostinil (weight of treprostinil is based on free treprostinil not its derivatives) to dry resin weight to weight ratio may be from 1:10 to 10:1 or from 1:5 to 5:1 or from 1:3 to 3:1 or 1:2.5 to 2.5: 1 or from 1:2 to 2:1 or from 1:1.8 to 1.8:1 or from 1:1.6 to 1.6: 1 or from 1:1.5 to 1.5: 1 or from 1:1.4 to 1.4:1 or from 1:1.3 to 1.3:1 or 1:1.2 to 1.2:1 or 1:1.1 to 1.1:1 or a subrange or value within these ranges.

In some embodiments of the suspension formulation, treprostinil/ion exchange resin complexes may be formulated in such a way that upon dispersion in an aqueous suspension medium for oral administration the concentration of treprostinil would be from 0.1 to 20 mg/ml, from 0.2 to 15 mg/ml, from 0.5 to 10 mg/ml, or from about 1 mg/ml to about 5 mg/ml.

In some embodiments, an ion exchange resin may be a cholestyramine resin, such as DUOLITE^{™} or PUROLITE^{™} resin, and treprostinil/ion exchange resin complexes may be formulated in such a way that upon dispersion in an aqueous media the concentration of treprostinil would be from 0.1 to 100 mg/ml or from 0.2 to 90 mg/ml or from 0.3 to 80 mg/ml or from 0.5 mg/ml to 70 mg/ml or from 1 mg/ml to 50mg/ml or a range within these subranges.

In some embodiments, the drug release from treprostinil-ion exchange resin compositions may be further prolonged or modified by treating the treprostinil-ion exchange resin complex with a release retardant. The release retardant may comprise a water-insoluble polymer or a combination of water-insoluble polymers.

In some embodiments, the release retardant does not form a separate layer on the treprostinil-ion exchange resin complex, but forms a matrix therewith. Examples of suitable release retardants include, for example, a polyvinyl acetate polymer or a mixture of polymers containing same (e.g., KOLLICOAT SR 30D), cellulose acetates, ethylcellulose polymers acrylic based polymers or copolymers (e.g., represented by the EUDRAGIT family of acrylic resins), cellulose phthalate, or a combination of such water-insoluble polymers or polymer systems, which are all "release retardants" as that term is used herein. These retardants may further prolong or alter the release of the treprostinil from the treprostinil-ion exchange resin complex and maximize attaining the desired release profile. Further, in some cases, the use of a release retardant may allow lowering the amount of coating thickness needed to attain a prolonged drug release, which may be for example, up to 24 hours. These retardants can be used in either substantially pure form or as a commercial preparation obtained from a vendor. Use of release retardants is disclosed for example, in U.S. Patent No. 8,597,684.

In some embodiments, the treprostinil-ion exchange resin complex may include a coating. In other words, in some embodiments, particles of the treprostinil-ion exchange resin complexes may be coated. In some embodiments, such coating may be a water insoluble coating. In other embodiments, the coating may be a polymer coating (i.e. a coating comprising one or more polymers), preferably, a water insoluble polymer coating (i.e. a coating comprising one or more water insoluble polymers). Examples of polymers, which may be used for the coating, include, but are not limited to, polyvinyl acetate, cellulose acetate, ethylcellulose polymers (such as Surerelease), cellulose phtatalate, hypromellose, ethyl acrylate, methyl methacrylate copolymers, acrylic acid, methacrylic acid copolymers, ethoxyethyl methacrylates, cyanoethyl methacrylates, polyacrylic acid, polymethacrylic acid, methacrylic acid alkylamide copolymers, polymethyl methacrylate, polymethacrylate, polyacrylamide, aminoalkyl methacrylate copolymers, polymethacrylic acid anhydride, glycidyl methacrylate copolymer, polyvinyl acetate, polyvinyl pyrrolidone, and polystyrene. In certain embodiments, a coating comprising cellulose acetate may be used.

Methods of making and applying polymer coatings are known in the art. In some embodiments, the coating may be deposited by spraying a solution or suspension comprising coating polymer on treprostinil/ion-exchange resin complexes. During such spraying, the treprostinil/ion-exchange resin complexes may be suspended in a fluidized bed, such as a wurster column.

In certain embodiments, the coating layer may be from 0.5% to 200%, 1 % to 150 %, from 1.5% to 100 %, from 2.0% to 75%, from 2.5 to 50 %, from 3 % to 30 %, or a subrange within these ranges, by weight, of the uncoated treprostinil-ion exchange resin complex. In some embodiments, the coating may further comprise a plasticizer in addition to one or more water insoluble polymers. The plasticizer can facilitate uniform coating of the treprostinil-ion exchange resin complex and/or enhance the tensile strength of the barrier coating layer. Suitable plasticizers are water soluble and water insoluble. Examples of suitable plasticizers include, e.g., dibutyl sebacate, propylene glycol, polyethylene glycol, polyvinyl alcohol, triethyl citrate, acetyl triethyl citrate, acetyl tributyl citrate, tributyl citrate, triacetin, Soluphor P, and mixtures thereof. Other plasticizers are described in U.S. Published Patent Application No. 2003/0099711 A1.

Where the coating comprises a cellulose acetate polymer, such polymer may be in an amount of 10 % to 99 %, from 30 % to 95 %, from 40 % to 90 %, from 50 to 90 % by weight, or a subrange within these ranges of the final coating.

The release rate of the coated treprostinil-ion exchange resin complexes that are designed as orally ingestible pharmaceutical compositions (such as liquid suspension, tablets, caplets, etc.) may be tailored to provide a desired drug release profile over a period of 1 to 36 hours, from 2 hours to 30 hours, from 2 hours to 24 hours, from 4 to 24 hours, from 6 to 24 hours, from 8 to 24 hours, or a subrange within these ranges.

This programmable release rate may be controlled principally by at least one of two variables - (1) the thickness of the polymer coating and (2) the use of a release retardant component, as described above, added to the treprostinil-ion exchange resin complex to form a fine particulate matrix prior to the polymer film coating step.

Treprostinil-ion exchange resin complexes may be used for treating a condition, for which treprostinil is known to be useful, by administering a therapeutically effective amount of a composition comprising a treprostinil-ion exchange ion complex to a subject, such as a human being, in need thereof. Such conditions include pulmonary hypertension, peripheral vascular disease, including intermittent claudication, ischemic lesions, critical limb ischemia, diabetic neuropathic foot ulcers, renal failure. In one embodiment, a composition comprising a treprostinil-ion exchange ion complex is administered to a subject to treat pulmonary arterial hypertension, including patients with NYHA Class II-IV PAH symptoms and patients with WHO functional class II-III symptoms and etiologies of PAH

Treprostinil-ion exchange resin complexes may readily be formulated with pharmaceutically acceptable excipients according to methods well known to those of skill in the art. In some embodiments, a formulation may contain only uncoated treprostinil-ion exchange resin complexes (i.e. such formulation does not contain coated treprostinil-ion exchange resin complexes). In some embodiments, a formulation may contain only coated treprostinil-ion exchange resin complexes (i.e. such formulation does not contain uncoated treprostinil-ion exchange resin complexes). Yet in some embodiments, a formulation may contain a mixture of coated and uncoated treprostinil-ion exchange resin complexes. In such mixture formulations, a weight ratio between coated and uncoated treprostinil-ion exchange resin complexes may vary. In some embodiments, the weight ratio between coated and uncoated treprostinil-ion exchange resin complexes may be from 100:1 to 1:100, from 50:1 to 1:50, from 20:1 to 1:20, from 10:1 to 1:10, from 5:1 to 1:5, from 2:1 to 1:2, or any subrange within these ranges.

Treprostinil ion-exchange resin complexes may be formulated for delivery by a suitable route including, orally, topically, intraperitoneally, transdermally, sublingually, intramuscularly, rectally, transbuccally, intranasally, liposomally, via inhalation, vaginally, intraoccularly, via local delivery (for example, by catheter or stent), subcutaneously, intraadiposally, intraarticularly, intravenously, or intrathecally.

The treprostinil-ion exchange resin composition thus prepared may be stored for future use or promptly formulated with conventional pharmaceutically acceptable carriers to prepare finished ingestible compositions for delivery orally, nasogastric tube, or via other means. The compositions may, for example, take the form of liquid preparations such as suspensions, or solid preparations such as capsules, tablets, caplets, sublinguals, powders, wafers, strips, gels, including liquigels, etc. In one embodiment, a tablet may be formulated as an orally disintegrating tablet. Such orally dissolving tablets may disintegrate in the mouth in less than about 60 seconds.

In some embodiments, the treprostinil-ion exchange resin composition may be formulated as a solid dosage form, such as a pill, a tablet, a capsule, a caplet, or a powder. In some embodiments, the treprostinil-ion exchange resin formulation may be formulated as an oral liquid dosage form, such as a solution, a syrup, a suspension, an elixir, a concentrate, an emulsion, or a dispersion.

The treprostinil-ion exchange resin compositions may be formulated using conventional pharmaceutically acceptable carriers or excipients and well-established techniques. Without being limited thereto, such conventional carriers or excipients include diluents, binders and adhesives (e.g., cellulose derivatives and acrylic derivatives), lubricants (e.g., magnesium or calcium stearate, or vegetable oils, polyethylene glycols, talc, sodium lauryl sulfate, polyoxy ethylene monostearate), thickeners, solubilizers, humectants, disintegrants, colorants, flavorings, stabilizing agents, sweeteners, and miscellaneous materials such as buffers and adsorbents in order to prepare a particular pharmaceutical composition. The stabilizing agents may include preservatives and anti-oxidants, as well as other components, which will be readily apparent to one of ordinary skill in the art.

Suitable thickeners include, e.g., tragacanth, xanthan gum, bentonite, starch, acacia and lower alkyl ethers of cellulose (including the hydroxy and carboxy derivatives of the cellulose ethers). Examples of cellulose include, e.g., hydroxypropyl cellulose, hydroxypropyl methyl cellulose, sodium carboxy methylcellulose, microcrystalline cellulose (MCC), and MCC with sodium carboxyl methyl cellulose. In one embodiment, tragacanth is used and incorporated in an amount of from about 0.1 to about 1.0% weight per volume (w/v) of the composition, and more preferably about 0.5% w/v of the composition. In another embodiment, xanthan gum is used in the amount of from about 0.025 to about 0.5% w/v and preferably about 0.25% w/v.

The treprostinil-ion exchange resin compositions may include a humectant composition to give the liquid greater viscosity and stability. Suitable humectants useful in the finished formulations include glycerin, polyethylene glycol, propylene glycol and mixtures thereof.

The oral liquid compositions of the present invention may also comprise one or more surfactants in amounts of up to about 5.0% w/v and preferably from about 0.02% w/v to about 3.0% w/v of the total formulation. The surfactants useful in the preparation of the finished compositions of the present invention are generally organic materials that aid in the stabilization and dispersion of the ingredients in aqueous systems for a suitable homogenous composition. Preferably, the surfactants of choice are non-ionic surfactants such as poly(oxyethylene)(20) sorbitan monooleate and sorbitan monooleate. These are commercially known as TWEENS and SPANS and are produced in a wide variety of structures and molecular weights.

Suitable polysorbates include polysorbate 20, polysorbate 40, polysorbate 80 and mixtures thereof. Most preferably, polysorbate 80 is employed. The surfactant component may comprise from about 0.01 to about 2.0% w/v of the total composition and preferably may comprise about 0.1% w/v of the total weight of the composition.

A second emulsifer/surfactant useful in combination with polysorbates may be employed , which may be a poloxamer such as Poloxamer 407. Poloxamer 407 has an HLB (hydrophilic/lipophilic balance) of about 22 and is sold under the tradename Pluoronic-127 (BASF--NJ). The two surfactants may be employed in substantially equivalent amounts. For example, the Poloxamer 407 and polysorbate 80 may each be employed together at levels of approximately from about 0.02 to about 4.0% w/v of the total weight of the formulation.

Aqueous suspensions may be obtained by dispersing the treprostinil-ion exchange resin compositions in a suitable aqueous vehicle, optionally with the addition of suitable viscosity enhancing agent(s) (e.g., cellulose derivatives, xanthan gum, etc). Non-aqueous suspensions may be obtained by dispersing the foregoing compositions in a suitable non-aqueous based vehicle, optionally with the addition of suitable viscosity enhancing agent(s) (e.g., hydrogenated edible fats, aluminum state, etc.). Suitable non-aqueous vehicles include, for example, almond oil, arachis oil, soybean oil or soybean oil or fractionated vegetable oils, such as fractionated coconut oil.

Useful preservatives include, but are not limited to, sodium benzoate, benzoic acid, potassium sorbate, salts of edetate (also known as salts of ethylenediaminetetraacetic acid, or EDTA, such as disodium EDTA), parabens (e.g., methyl, ethyl, propyl or butyl-hydroxybenzoates, etc.), and sorbic acid. Amongst useful preservatives include chelating agents some of which are listed above and other chelating agents, e.g., nitrilotriacetic acid (NTA), ethylenediaminetetracetic acid (EDTA), hydroxyethylethylenediaminetriacetic acid (HEDTA), diethylenetriaminepentaacetic acid (DPTA), 1,2-Diaminopropanetetraacetic acid (1,2-PDTA), 1,3-Diaminopropanetetraacetic acid (1,3-PDTA), 2,2-ethylenedioxybis[ethyliminodi(acetic acid)] (EGTA), 1,10-bis(2-pyridylmethyl)-1,4,7,10-tetraazadecane (BPTETA); ethylenediamine (EDAMINE), Trans-1,2-diaminocyclohexane-N,N,N',N'-tetraacetic acid (CDTA); ethylenediamine-N,N'-diacetate (EDDA), phenazine methosulphate (PMS); 2,6-Dichloro-indophenol (DCPIP), Bis(carboxymethyl)diaza-18-crown-6 (CROWN), porphine, chlorophyll, dimercaprol (2,3-Dimercapto-1-propanol), citric acid, tartaric acid, fumaric acid, malic acid, and salts thereof. The preservatives listed above are exemplary, but each preservative must be evaluated in each formulation, to assure the compatibility and efficacy of the preservative. Methods for evaluating the efficacy of preservatives in pharmaceutical formulations are known to those skilled in the art. Preferred preservatives are the paraben preservatives include, methyl, ethyl, propyl, and butyl paraben. Methyl and propyl paraben are most preferable. Preferably, both methyl and propyl paraben are present in the formulation in a ratio of methyl paraben to propyl paraben of from about 2.5:1 to about 16:1, and in some embodiments, 9:1.

In the instance where auxiliary sweeteners are utilized, it may be contemplated the inclusion of those sweeteners well known in the art, including both natural and artificial sweeteners. Thus, additional sweeteners may be chosen from the following non-limiting list: water-soluble sweetening agents such as monosaccharides; disaccharides and polysaccharides such as xylose, ribose, glucose, mannose, galactose, fructose, high fructose corn syrup, dextrose, sucrose, sugar, maltose, partially hydrolyzed starch, or corn syrup solids; and sugar alcohols such as sorbitol, xylitol, mannitol and mixtures thereof.

In general, the amount of sweetener will vary with the desired amount of sweeteners selected for a particular liquid formulation. This amount will normally be 0.001 to about 90% by weight, per volume of the final liquid composition, when using an easily extractable sweetener. The water-soluble sweeteners described above, are preferably used in amounts of about 5% to about 70% by weight per volume, and most preferably from about 10% to about 50% by weight per volume of the final liquid composition. In contrast, the artificial sweeteners (e.g., sucralose, acesulfame K, and dipeptide based sweeteners) are used in amounts of about 0.005% to about 5.0% and most preferably about 0.01% to about 2.5% by weight per volume of the final liquid composition. These amounts are ordinarily necessary to achieve a desired level of sweetness independent from the flavor level achieved from flavor oils.

Suitable flavorings include both natural and artificial flavors, and mints such as peppermint, menthol, artificial vanilla, cinnamon, various fruit flavors, both individual and mixed, essential oils (e.g., thymol, eculyptol, menthol and methyl salicylate) and the like are contemplated. The amount of flavoring employed is normally a matter of preference subject to such factors as flavor type, individual flavor, and strength desired. Thus, the amount may be varied in order to obtain the result desired in the final product. Such variations are within the capabilities of those skilled in the art without the need for undue experimentation. The flavorings are generally utilized in amounts that will vary depending upon the individual flavor, and may, for example, range in amounts of about 0.01% to about 3% by weight per volume of the final composition weight.

Useful colorants, include the pigments such as titanium dioxide, that may be incorporated in amounts of up to about 1% by weight per volume, and preferably up to about 0.6% by weight per volume. Also, the colorants may include dyes suitable for food, drug and cosmetic applications, and known as D&C and F.D. & C. dyes and the like. The materials acceptable for the foregoing spectrum of use are preferably water-soluble. Illustrative examples include indigoid dye, known as F.D. & C. Blue No. 2, which is the disodium salt of 5,5' indigotindisulfonic acid. Similarly, the dye known as F.D. & C. Green No. 1 comprises a triphenylmethane dye and is the monosodium salt of 4-[4-N-ethyl p-sulfobenzylamino)diphenylmethylene]-[1-(N-ethyl-N-p-sulfoniumbenzyl)-2,-5-cyclohexadienimine]. A full recitation of all F.D. & C. and D. & C. and their corresponding chemical structures may be found in the Kirk-Othmer Encyclopedia of Chemical Technology, in Volume 5, at Pages 857-884, which text is incorporated herein by reference.

Suitable oils and fats that are usable would include partially hydrogenated vegetable or animal fats, such as coconut oil, palm kernel oil, beef tallow, lard, and the like. These ingredients are generally utilized in amounts with respect to the comestible product of up to about 7.0% by weight, and preferably up to about 3.5% by weight of the final product.

Wetting agents also may be employed in the compositions to facilitate the dispersion of any hydrophobic ingredients. The concentration of wetting agents in the composition should be selected to achieve optimum dispersion of the ingredient within the composition with the lowest feasible concentration of wetting agent. It should be appreciated that an excess concentration of wetting agent may cause the composition, as a suspension, to flocculate. Those skilled in the art are well versed in suitable empirical methods to determine the appropriate wetting agents and concentrations to achieve optimum dispersion and avoid flocculation. Suitable wetting agents are listed in the U.S. Pharmacoepia 29.

Embodiments described herein are further illustrated by the following working examples.

### Example

### Introduction

A study was performed to develop a sustained-release liquid dosage form of treprostinil, in which the rate of release of the drug (treprostinil) may be controlled. In this study, the complex between treprostinil and an ion-exchange resins (IER), namely a cholestyramine resin, was characterized as a candidate for use in a sustained-release dosage form of treprostinil.

### Materials

Chemical formulae of treprostinil diolamine (UT-15C) and treprostinil (UT-15) are as follows: 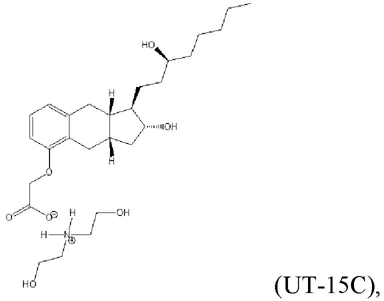 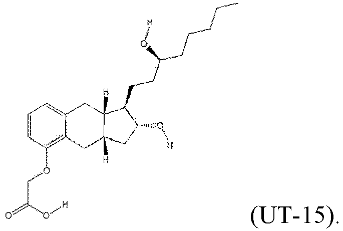 In this study, the following materials were used: 1) treprostinil diolamine (UT-15C) Batch number: D02D11017; 2) PUROLITE^{™} A430MR cholestyramine resin, Batch number: 2009Y/14/5, kindly provided by Purolite SRL (Brasov, Romania); and 3) DUOLITE^{™} AP143/1083 Cholestyramine Resin USP, Batch number: A075DBL013, kindly provided by Dow Chemical Company (Chauny, France).

### Methods

A UV absorbance measurement was conducted with an approximate 0.1 mg/mL treprostinil diolamine solution with a Fluostar Omega BMG Labtech UV spectrophotometer from 220 nm to 350 nm to determine the λₘₐₓ. Two standard curves were established with known concentrations of treprostinil at 228 nm and 270 nm. The time to achieve equilibrium between bound and unbound treprostinil to the IER was determined. One gram of resin was added to 100 mL of 10 mg/mL treprostinil solution. The suspension was stirred for four hours and 1-mL samples were taken at 30, 60, 90, 120, 180, and 240 minutes. Each sample was filtered through a 0.45-µm PTFE filter, and the resulting solution was assayed by UV spectrophotometry for treprostinil concentration. Complexation experiments were conducted with known concentrations of treprostinil and one gram of resin at ratios of 1:0.5, 1:1, 1:2, and 1:4, resin:treprostinil (w/w). Two resins, PUROLITE^{™} and DUOLITE^{™}, were evaluated separately. The amount of treprostinil complexed per gram of resin was calculated based on the difference between the initial and final treprostinil concentration. The percent loading efficiency was calculated based on the equation: (1-[unbound treprostinil]/[initial treprostinil])∗100. Photographs were taken for each resin before and after complexation with treprostinil using a Leica DM IL LED light microscope and Leica DMC2900 microscope camera.

### Results

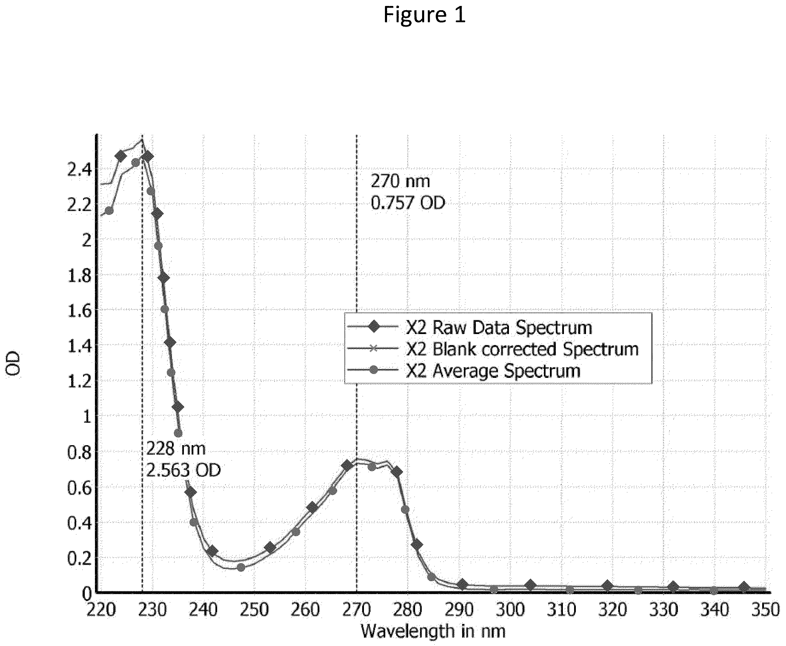

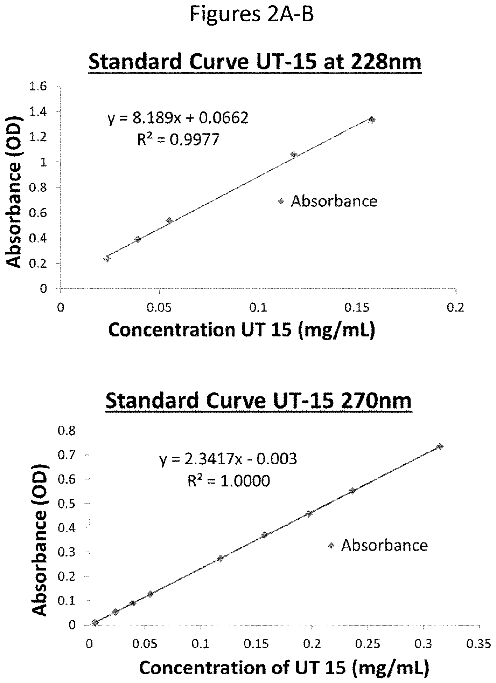

Figures 1-6 present the results of experiments. In particular, Figure 1 shows a UV absorbance spectrum of treprostinil diethanolamine, which has maxima at 228 nm and 270 nm. Figures 2A and 2B show calibration curves for determining a concentration of unbound treprostinil (diethanolamine) based on treprostinil absorbance at 228 nm (2A) and 270 nm (2B). The 270 nm curve was used to calculate all the concentrations in the complexation experiments.

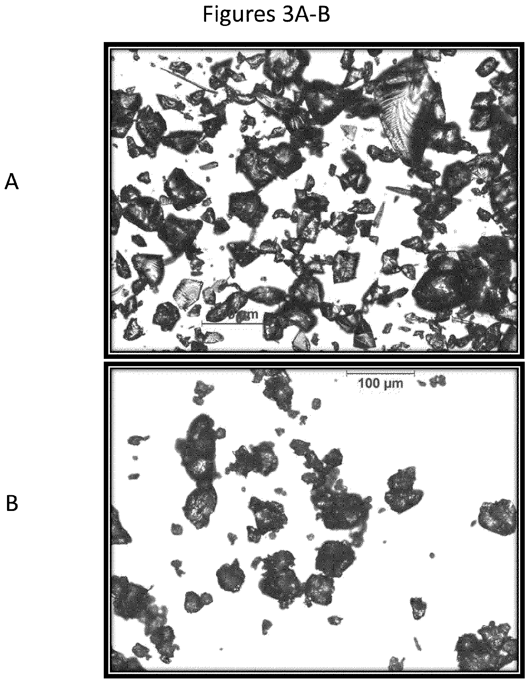

Figures 3A and 3B show respectively photographs of PUROLITE^{™} and DUOLITE^{™} resins before complexation.

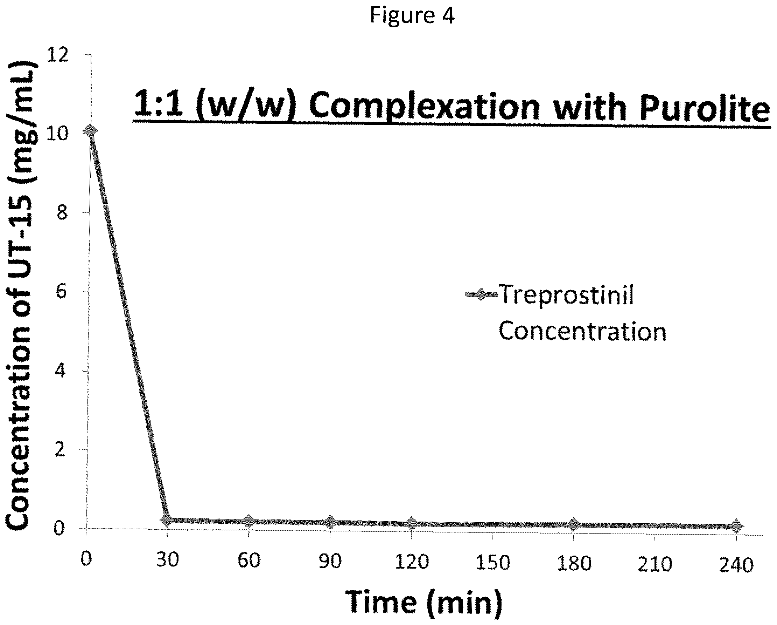

Figure 4 is a graph showing a concentration of unbound treprostinil when treprostinil is mixed with PUROLITE^{™} resin using a treprostinil:resin ratio of 1:1 (w/w) as a function of time. Figure 4 shows that the concentration of unbound treprostinil decreases significantly before reaching equilibrium.

Treprostinil diethanolamine was determined to be stable for 14 days at both ambient and refrigerated environments. Treprostinil diethanolamine was also shown to have no binding effect with the 0.45-µm polytetrafluoroethylene (PTFE) filters used in the assay.

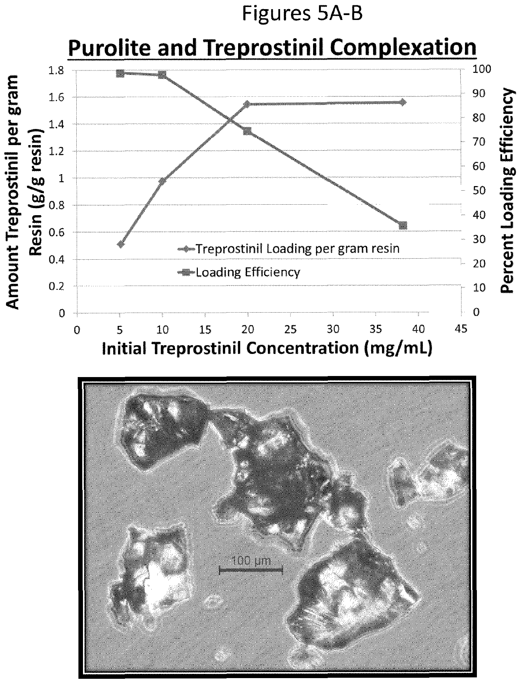

Figure 5A is a graph showing i) treprostinil diolamine loading per gram resin and ii) treprostinil diethanolamine loading efficiency during PUROLITE^{™} resin and treprostinil diolamine complexation. Figure 5B is a photograph of PUROLITE^{™} and treprostinil diolamine complexes.

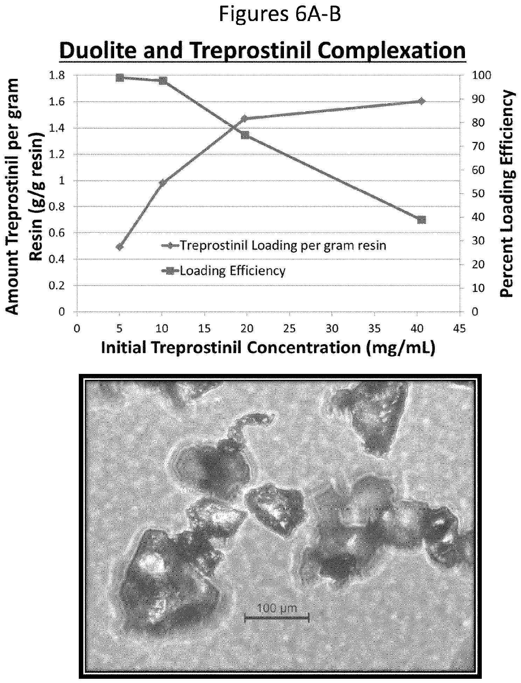

Figure 6A is a graph showing i) treprostinil loading per gram resin and ii) treprostinil loading efficiency during DUOLITE^{™} resin and treprostinil complexation. Figure 6B is a photograph of DUOLITE^{™} and treprostinil complexes.

### Conclusions

Treprostinil has λₘₐₓ at wavelengths of 228 nm and 270 nm. Treprostinil forms an ionic complex with cholestyramine resins. After one hour of stirring, the equilibrium between bound and unbound treprostinil to the resins was achieved. With an increasing concentration of treprostinil diethanolamine, more drug was complexed to the resin, but started to plateau where the maximum amount of treprostinil bound reached 1.6 g / g resin. With lower concentrations of treprostinil diethanolamine, the loading was more efficient where almost all drug was bound to the resin. A preferred ratio for complexation may be 1:1, resin:drug, where the most drug can be bound, with the least amount of waste.

### Additional Literature

Pande S.V., et al. International Journal of Advances in Pharmaceutical Sciences 2. 2.1 (2011): 8-16. Print.

## Claims

1. A composition comprising a) treprostinil or a pharmaceutically acceptable salt therof and b) an anion ion-exchange resin.

2. The composition of claim 1, wherein the treprostinil or salt therof forms an ion complex with the anion exchange resin.

3. The composition of claim 1, wherein the ion-exchange resin comprises a bile acid sequestrant.

4. The composition according to claim 3, wherein the bile acid sequestrant is selected from the group consisting of cholestyramine, colosevelam, and colestipol.

5. The composition of claim 4, wherein the ion-exchange resin is a cholestyramine resin.

6. The composition of claim 5, wherein a weight-to-weight ratio between treprostinil and the cholestyramine resin is from 1:2 to 2:1.

7. The composition of claim 5, prepared using an aqueous dispersion of treprostinil and the cholestyramine resin, wherein the dispersion has a concentration of treprostinil from 0.1 mg/ml to 100 mg/ml.

8. A pharmaceutical formulation comprising i) an ion complex formed between treprostinil or a pharmaceutically acceptable salt therof and an anion ion-exchange resin and ii) a pharmaceutically acceptable carrier.

9. The formulation of claim 8, wherein the formulation is (i) a suspension; (ii) a solid dosage form selected from tablets and capsules or (iii) a liquid dosage form for oral delivery.

10. The formulation of claim 8, further comprising a water insoluble membrane coating on the ion complex.

11. The formulation of claim 10, wherein said coating comprises a polymer or wherein said coating comprises cellulose acetate.

12. The formulation of claim 8, wherein said formulation further comprises a release retardant configured to prolong or modify a release of the treprostinil from the ion complex.

13. The formulation of claim 8, wherein the formulation is a controlled-release formulation providing a controlled release of the treprostinil over a time period ranging from 1 hour to 36 hours.

14. A formulation according to claim 8, for use in a method of treating pulmonary hypertension comprising administering to a subject in need thereof a therapeutically effective amount of the formulation.

15. A method of preparing a treprostinil formulation comprising admixing an ion-exchange resin and a solution comprising treprostinil or a pharmaceutically acceptable salt therof to form a suspension comprising an ion complex of treprostinil or a pharmaceutically acceptable salt therof and the ion-exchange resin.

## Patentansprüche

1. Zusammensetzung, umfassend a) Treprostinil oder ein pharmazeutisch annehmbares Salz hiervon und b) ein Anionen-Ionenaustauschharz.

2. Zusammensetzung gemäß Anspruch 1, worin das Treprostinil oder das Salz hiervon einen Ionenkomplex mit dem Anionen-Austauschharz bildet.

3. Zusammensetzung gemäß Anspruch 1, worin das IonenAustauschharz ein Gallensäure-Sequestriermittel umfasst.

4. Zusammensetzung gemäß Anspruch 3, worin das Gallensäure-Sequestriermittel ausgewählt ist aus der Gruppe bestehend aus Cholestyramin, Colosevelam und Colestipol.

5. Zusammensetzung gemäß Anspruch 4, worin das IonenAustauschharz ein Cholestyraminharz ist.

6. Zusammensetzung gemäß Anspruch 5, worin das Gewichtsverhältnis zwischen Treprostinil und dem Cholestyraminharz von 1:2 bis 2:1 beträgt.

7. Zusammensetzung gemäß Anspruch 5, zubereitet unter Verwendung einer wässrigen Dispersion von Treprostinil und des Cholestyraminharzes, worin die Dispersion eine Treprostinilkonzentration von 0,1 mg/ml bis 100 mg/ml aufweist.

8. Pharmazeutische Formulierung, umfassend i) einen Ionenkomplex, gebildet zwischen Treprostinil oder einem pharmazeutisch annehmbaren Salz hiervon und einem Anionen-Ionenaustauschharz und ii) einen pharmazeutisch annehmbaren Träger.

9. Formulierung gemäß Anspruch 8, worin die Formulierung (i) eine Suspension; (ii) eine feste Dosierungsform, ausgewählt aus Tabletten und Kapseln, oder (iii) eine flüssige Dosierungsform für die orale Verabreichung ist.

10. Formulierung gemäß Anspruch 8, ferner umfassend eine wasserunlösliche Membran, die den Ionenkomplex beschichtet.

11. Formulierung gemäß Anspruch 10, worin die Beschichtung ein Polymer umfasst, oder worin die Beschichtung Celluloseacetat umfasst.

12. Formulierung gemäß Anspruch 8, worin die Formulierung ferner ein Freisetzungs-Verzögerungsmittel umfasst, das konfiguriert ist, um die Freisetzung des Treprostinils aus dem Ionenkomplex zu verlängern oder zu modifizieren.

13. Formulierung gemäß Anspruch 8, worin die Formulierung eine Formulierung mit kontrollierter Freisetzung ist, die eine kontrollierte Freisetzung des Treprostinils über eine Zeitspanne im Bereich von 1 bis 36 Stunden bereitstellt.

14. Formulierung gemäß Anspruch 8 zu Verwendung in einem Verfahren zur Behandlung von pulmonarer Hypertonie, umfassend die Verabreichung einer therapeutisch wirksamen Menge der Formulierung an einen Patienten, der dessen bedarf.

15. Verfahren zur Herstellung einer Treprostinilformulierung, umfassend Vermischen eines Ionen-Austauschharzes und einer Lösung, die Treprostinil oder ein pharmazeutisch annehmbares Salz hiervon umfasst, um eine Suspension zu bilden, die einen Ionenkomplex von Treprostinil oder einem pharmazeutisch annehmbaren Salz hiervon und dem IonenAustauschharz umfasst.

## Revendications

1. Composition comprenant a) du tréprostinil ou un sel pharmaceutiquement acceptable de celui-ci et b) une résine échangeuse d'ions anioniques.

2. Composition selon la revendication 1, dans laquelle le tréprostinil ou le sel de celui-ci forme un complexe ionique avec la résine échangeuse d'anions.

3. Composition selon la revendication 1, dans laquelle la résine échangeuse d'ions comprend un séquestrant d'acides biliaires.

4. Composition selon la revendication 3, dans laquelle le séquestrant d'acides biliaires est sélectionné à partir du groupe consistant en la cholestyramine, le colosevelam et le colestipol.

5. Composition selon la revendication 4, dans laquelle la résine échangeuse d'ions est une résine de cholestyramine.

6. Composition selon la revendication 5, dans laquelle un rapport poids sur poids entre le tréprostinil et la résine de cholestyramine va de 1:2 à 2:1.

7. Composition selon la revendication 5, préparée à l'aide d'une dispersion aqueuse de tréprostinil et de la résine de cholestyramine, dans laquelle la dispersion présente une concentration de tréprostinil allant de 0,1 mg/ml à 100 mg/ml.

8. Formulation pharmaceutique comprenant i) un complexe ionique formé entre le tréprostinil ou un seul pharmaceutiquement acceptable de celui-ci et une résine échangeuse d'ions anioniques et ii) un vecteur pharmaceutiquement acceptable.

9. Formulation selon la revendication 8, dans laquelle la formulation est (i) une suspension ; (ii) une forme posologique solide sélectionnée parmi des comprimés et des capsules ou (iii) une forme posologique liquide pour administration orale.

10. Formulation selon la revendication 8, comprenant en outre un enrobage à membrane insoluble dans l'eau sur le complexe ionique.

11. Formulation selon la revendication 10, dans laquelle ledit enrobage comprend un polymère ou dans laquelle ledit enrobage comprend de l'acétate de cellulose.

12. Formulation selon la revendication 8, dans laquelle ladite formulation comprend en outre un retardateur de libération conçu pour prolonger ou modifier une libération du tréprostinil depuis le complexe ionique.

13. Formulation selon la revendication 8, dans laquelle la formulation est une formulation à libération régulée fournissant une libération régulée du tréprostinil sur une période de temps allant de 1 heure à 36 heures.

14. Formulation selon la revendication 8, pour utilisation dans un procédé de traitement d'hypertension pulmonaire comprenant l'administration à un sujet en ayant besoin d'une quantité thérapeutiquement efficace de la formulation.

15. Procédé de préparation d'une formulation de tréprostinil comprenant le mélange d'une résine échangeuse d'ions et d'une solution comprenant du tréprostinil ou un sel pharmaceutiquement acceptable de celui-ci pour former une suspension comprenant un complexe ionique de tréprostinil ou d'un sel pharmaceutiquement acceptable de celui-ci et de la résine échangeuse d'ions.
